Europäisches Patentamt

European Patent Office

Office européen des brevets

(19)

(11) Publication number: **0 172 716**
**A1**

(12) # EUROPEAN PATENT APPLICATION

(21) Application number: **85305707.3**

(22) Date of filing: **12.08.85**

(51) Int. Cl.⁴: **A 61 L 27/00**

(30) Priority: **14.08.84 US 640726**
**26.11.84 US 674897**

(43) Date of publication of application:
**26.02.86 Bulletin 86/9**

(84) Designated Contracting States:
**BE CH DE FR GB IT LI LU NL SE**

(71) Applicant: **SHILEY INCORPORATED**
**17600 Gilette**
**Irvine California(US)**

(72) Inventor: **Torrianni, Mark W.**
**1742, Preluda Drive Anaheim**
**Orange County California(US)**

(72) Inventor: **Zadro, Rebecca K.**
**9641, Dove Circle Fountain Valley**
**Orange County California(US)**

(74) Representative: **Wood, David John et al,**
**Pfizer Limited Ramsgate Road**
**Sandwich Kent CT13 9NJ(GB)**

(54) **Retarded mineralization of implantable tissue by surface active betaines.**

(57) Mineralization of implantable tissue, such as pericardial tissue for heart valve manufacture, is retarded by immersion of the tissue in an aqueous solution of a surface active betaine before fixation of the tissue.

EP 0 172 716 A1

0172716

P.C.(HOSH)   6852A

The invention relates to a process for preparing implantable tissue in which mineralization is retarded after implantation, and to implantable tissue which is so prepared.

Natural tissue taken from animals including humans has been used in the past for the preparation of prosthetic device for use in humans. For instance, heart valves made from porcine aortic roots are described in U.S. Patent No. 3,966,401. Prostheses in the form of tubes, patches and conduits may be prepared from arteries and veins of umbilical cords according to U.S. Patent No. 3,988,782. Numerous stabilization methods to increase the longevity of the natural tissue in the host body are known. Commonly the treatment is with a tanning agent such as glutaraldehyde as disclosed in U.S. Patent No. 4,378,224.

Mineralization of bioprosthetic heart valves after implantation has been encountered resulting in reduced flexibility of the valve tissue and therefore decreased efficiency in the operation of the prosthesis in the host body. One solution to this problem suggested by Lentz et al U.S. Patent No. 4,323,358 granted April 6, 1982 is treatment of the fixed tissue with a solution of a water soluble salt of a sulfated higher aliphatic alcohol, particularly sodium dodecyl sulfate (SDS). Although this treatment with an anion surfactant delays the onset of mineralization to some extent, further retardation is obtained with the amphoteric surfactant of the invention as well as inhibition of the rate of mineralization.

According to the invention, implantable tissue is prepared by immersing said tissue in an aqueous solution of a surface active betaine before fixation of the tissue.

The surfactant for use in the invention may be any surface-active betaine including surface active sulfobetaines, phosphobetaines and carboxyalkylbetaines. Particularly preferred surfactants are the sulfobetaines, e.g. N-decyl-N,N-dimethyl-3-ammonium-1-propanesulfonate (Zwittergent[R] 3-10 Detergent) and N-hexadecyl-N,N-dimethyl-3-ammonium-1-propanesulfonate (Zwittergent[R] 3-16 Detergent), described at page 279 of the 1983 Catalogue of Calbiochem-Behring for Research Biochemicals and Immuno-chemicals.

The tissue treated according to the invention is usually bovine or porcine pericardial tissue, although other tissue may be used such as dura mater, ligament, aortic roots, tendon, heart valve tissue, etc.

The unfixed, untreated natural tissue is first carefully cleaned while immersed in buffered saline solution. The buffer may be any suitable phosphate or calcium free buffer known in the art, e.g. acetate buffer. The salt is a water-soluble salt such as a salt of an alkali metal or alkaline earth metal and a halide such as a chloride. This type of solution is meant whenever general reference is made herein to a buffered saline solution.

More specifically, according to the invention, the tissue is immersed in an aqueous solution of from about 0.01 to 10, e.g. 0.01 to 2, % by weight of a surface active betaine. The aqueous solution is preferably a buffered solution of the type described above.

The tissue is generally immersed in the surfactant solution for at least one hour, e.g. 4 hours. Usually, immersion is for about 24 hours, although longer immersing is acceptable.

After immersing, the tissue can then be rinsed in a buffered saline solution and then fixed in glutaraldehyde (GA) for about 7 to 14 or more days. The GA-fixative is any fixative known in the art such as a buffered aqueous solution of about 0.01 to about 2% GA buffered at a pH from about 4.0 to 8.5, preferably from abut 4.5 to 6.5 and more preferably at 5.5.

After fixation, the tissue may be used in the manufacture of a prosthesis, such as a heart valve.

For final storage, the tissue is aseptically transferred to a phosphate-free buffered formaldehyde solution having a pH from abut 4.5 to 8.5.

The effectiveness of the treatments in retarding mineralization, specifically calcification, of the tissue was determined by animal implant tests according to the following procedure.

New Zealand white rabbits weighing about 1 to 3 kg were anesthetiszed and prepared for surgery. An 8-10 cm patch of skin located at the vertebral border of the scapula was cleared of hair. The shaved area and surrounding unshaved areas were sterilized. On either side of the spinal column a 4 cm incision was made to clear the dermis but not cut the underlying trapezius muscles. In the subdermal space a small pocket was made by forcing apart the connective facia. A 2x3 cm sterile sample of pericardial tissue rinsed three times with sterile saline was placed in one pocket and a 2x3 cm sterile sample of pericardial tissue treated with a surface active betaine and rinsed with sterile saline was placed in the other pocket. The incisions were closed and the animal returned to its cage.

After a prescribed length of time (Implant Time in the Table below), the animal was sacrificed and the original patch shorn again and rinsed to decrease the amount of hair around the surgical site. The implanted tissue was removed and placed in a 4% formaldehyde solution for calcium quantitation.

The calcium quantitation was done as follows. The implanted tissue was fixed in acetate-buffered 4% formaldehyde having a pH of 5.5. After dissection of histological samples, the remaining sections of the original 2x3 cm implant were dried in a vented oven at 50°C for 24 hours. The dried samples were weighed on an analytical balance accurate to 0.00001 g and then extracted in 3.0 ml of a 1:1 solution of 16 N nitric acid and 30% hydrogen peroxide at 23°C for 24 hours or until no tissue was visible. The extract was measured for calcium by atomic absorption spectrophotometry. The sensitivity of the Beckman 2380 Atomic Abbsorption spectrophotometer for $Ca^{++}$ was 0.092 ppm or less. The linear range for $Ca^{++}$ was 4.0 ppm to be assayed. The samples were also diluted with 0.2% lanthanum chloride to reduce the interference of elements that form stable oxygen salts with $Ca^{++}$.

The following example illustrates the invention.

### Example

Unfixed, untreated, natural bovine pericardial tissue was carefully cleaned while immersed in the buffered saline solution described below. The cleaned tissue was immersed in a 0.1% aqueous solution of N-hexadecyl-N,N-dimethyl-3-ammonium-1-propane sulfonate for 22 hours at 4°C. The zwitterionic detergent solution was prepared by dissolving 1.0 g of N-hexadecyl-N,N-dimethyl-3-ammonium-1-propane sulfonate in

sufficient of the buffered saline solution described below to yield one liter of 0.1% w/v (weight per volume) detergent solution.

The buffered saline solution was an 0.05 M acetate buffer containing 0.35% by weight sodium acetate and 0.4% by weight 2M acetic acid, as well as 0.05% by weight sodium chloride. The pH of the buffered saline solution was adjusted to 5.5 with 1N acetic acid.

The final detergent solution had a pH of 5.5.

After exposure to the detergent solution, the tissue was rinsed in the above buffered saline solution. The rinsed tissue was placed in 0.5% glutaraldehyde solution buffered with the above 0.05 M acetate buffer to a pH of 5.5 for fixation for 7 days.

The results of the above animal implant test are tabulated below. The pre-fixation sample is obtained in accordance with the above Example. The tests with tissue from Hancock T-6 are comparative samples showing that the prior art treatment is less effective in delaying onset of calcification than the treatment of the invention and less effective in inhibiting the rate of calcification. The Hancock heart valve tissue was taken from a commercially available Hancock Extracorporeal heart valve labeled as follows:

Hancock T6 Treated with Pericardial Heart Valve
Model: T 405. Size: 25mm.
Serial No.: A8682
Manufacturing Release Date: October 20, 1982.
The Hancock heart valve tissue had undergone the SDS post-fixation treatment of U.S. Patent 4,323,358.

TABLE

| Surfactant | Implant Time (days) | Ca$^{++}$ (ug/mg) (Dry weight) |
|---|---|---|
| Tissue from Hancock T-6 Valve | 60 | 0.23 |
| | 124 | 413.86 |
| | 187 | 418.38 |
| Zwittergent® 3-16 Detergent (pre-fixation) | 14 | 0.29 |
| Control | 14 | 14.64 |
| Sample | 30 | 0.36 |
| Control | 30 | sample not found |
| Sample | 56 | 0.24 |
| Control | 56 | 216.73 |
| Sample | 84 | 0.81 |
| Control | 84 | 165.83 |
| Sample | 182 | 101.16 |
| Control | 182 | 492.86 |

P.C. 6852/A

## CLAIMS

1. A process for preparing implantable tissue to retard mineralization characterized by immersing said tissue in an aqueous solution of a surface active betaine before fixing said tissue.

2. A process according to claim 1, characterized in that said tissue is tendon, heart valve tissue, dura mater, ligament or pericardium.

3. A process according to claim 1 or 2, characterized in that said fixing is with glutaraldehyde.

4. A process according to 3, characterized in that said fixing is with an aqueous solution of 0.01 to 2% by weight glutaraldehyde buffered at a pH from abut 4.5 to 8.5.

5. A process according to any one of Claims 1 to 4, characterized in that said aqueous solution contains from about 0.01 to 2% by weight of said surface active betaine.

6. A process according to any one of claims 1 to 5, characterized in that said tissue is contacted with said surfactant for at least about 4 hours.

7. A process according to any one of Claims 1 to 6, characterized in that said surface active betaine is N-hexadecyl-N,N-dimethyl-3-ammonium-1-propanesulfonate.

8. A process according to any one of Claims 1 to 7, characterized in that said tissue is washed in buffered saline after said immersing step and before storage or fixation in glutaraldehyde.

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| Y | WO-A-8 401 879 (AMERICAN HOSPITAL SUPPLY) * Claims 1,6-9,11-14,19,33,35 * | 1-8 | A 61 L 27/00 |
| Y | EP-A-0 050 061 (E. SHANBROM) * Page 5, lines 5-9; page 9, line 16 * | 1-8 | |
| A | EP-A-0 103 946 (EXTRACORPOREAL MEDICAL SPECIALITIES) | 1 | |
| A | EP-A-0 099 445 (NEW YORK BLOOD CENTER) * Page 10, lines 28-30 * | 1 | |

TECHNICAL FIELDS SEARCHED (Int. Cl.4)

A 61 L

The present search report has been drawn up for all claims

| Place of search THE HAGUE | Date of completion of the search 12-11-1985 | Examiner PELTRE CHR. |
|---|---|---|